# EUROPÄISCHE PATENTANMELDUNG

(11) **EP 0 598 412 A2**
(43) Veröffentlichungstag der Anmeldung: **25.05.1994**
(21) Anmeldenummer: 93118654.8
(22) Anmeldetag: 19.11.1993
(51) Int. Cl.: A61K 7/40, A61K 7/48

(54) **Hautschutzmittel zum Schutz der menschlichen Haut**

(30) Priorität: 19.11.1992 DE 4238860
(71) Anmelder: MEDICON GESELLSCHAFT FÜR UNTERNEHMENSBERATUNG IM BEREICH MEDIZIN UND GESUNDHEITSWESEN mbH, D-48282 Emsdetten (DE)
(72) Erfinder: Neubourg, Fritz, D-48268 Greven (DE)
(74) Vertreter: Spalthoff, Adolf, Dipl.-Ing.

(57) **Zusammenfassung**

Hautschutzmittel zum Schutze der menschlichen Haut gegen Austrocknung und aggressive, hautirritierende und allergisierende Substanzen sind in verschiedenen Darreichungsformen ausbildbar, z.B. als Gel, Lotion, Salbe, Creme, Schaum etc.
Zur Verbesserung der Verträglichkeits- und Schutzeigenschaften der genannten Hautschutzmittel wird vorgeschlagen, daß ihnen als Komponente Polytetrafluorethylen (PTFE), vorzugsweise mit einem Anteil von 0,01 - 12,00 Gew.-%, beigegeben wird.

## Beschreibung

Die Erfindung bezieht sich auf ein Hautschutzmittel zum Schutz der menschlichen Haut gegen Austrocknung und aggressive, hautirritierende und allergisierende Substanzen, welches in verschiedenen Darreichungsformen, z.B. als Gel, Lotion, Salbe, Creme, Schaum etc. ausbildbar ist.

Für verschiedene Berufstätige ist die durch die Berufstätigkeit selbst verursachte Hautbelastung vergleichsweise hoch. So kommen z.B. Friseure bei der Ausübung ihrer Tätigkeit mit Dauerwellflüssigkeiten, wässrigem Wasserstoffperoxyd, Tensiden und Haarfärbemitteln in Berührung und haben länger anhaltenden Kontakt mit Wasser und Umgang mit Scheren aus Nickel oder nickelhaltigen Legierungen. Industriearbeiter sind mit ihren Händen vergleichsweise häufig unvorhergesehen und schutzlos in Kontakt mit Säuren, Laugen, Ölen, Fetten, Bohrölen, Kühlschmiermitteln, Schneidmitteln, Farben, Lacken, Lösungsmitteln, Schmutz etc. Gärtner und Floristen haben bei der Ausübung ihrer Tätigkeit gelegentlich schutzlos Kontakt mit Herbiziden, Insektiziden und Pflanzeninhaltsstoffen oder allergisierend wirksamen Pflanzenteilen. Die Ausübenden medizinischer Berufe sind durch häufiges Händewaschen, Kontakt mit Alkohol, Äther, Desinfektionsmitteln, Mazeration der Haut durch Feuchtigkeitskammern sowie durch Benutzung von Talkpuder in den Gummihandschuhen gefährdet. Bei Arbeitnehmern im Nahrungsmittelgewerbe, z.B. bei Bäckern, hat sich der Umgang mit Mehl als Belastung für die Haut erwiesen. Bei Arbeitnehmern im Baugewerbe äußert sich die Belastung z.B. in der häufigen Berührung mit im Zement enthaltenen Chromaten.

Der Erfindung liegt die Aufgabe zugrunde, ein Hautschutzmittel zur Verfügung zu stellen, mittels dem die Hautbelastung für den vorstehend genannten Personenkreis sowie für andere, entsprechend belastete Personenkreise verringert werden kann. Die Schutzwirkung derartiger galenischer Hautschutzmittel gegen aggressive, hautirritierende und allergisierende Substanzen oder gegen Schadstoffe aus der Umgebungsluft soll so verbessert werden, daß nach der Applikation der Hautschutzmittel auf die Haut sowohl im alkalischen als auch im sauren Bereich eine ausgeprägte Pufferkapazität erreicht wird, durch welche die Haut gegenüber den genannten schädlichen Einflüssen im sauren oder alkalischen Milieu ausreichend geschützt ist. Insbesondere soll die hautreizende Wirkung von Chemikalien, Schadstoffen aus der Umgebungsluft sowie von allergisierend wirkenden Metallen, wie z.B. Nickel und/oder nickelhaltigen Legierungen, abgeschwächt bzw. neutralisiert werden. Außerdem soll durch die Zubereitungen ein Schutz vor der hautschädigenden Wirkung von Wasser bei länger andauernder Exposition während der Arbeiten im Beruf oder im Haushalt und beim Tragen von Gummihandschuhen, die als Feuchtigkeitskammer wirken, erreicht werden.

Diese Aufgabe wird erfindungsgemäß dadurch gelöst, daß das Hautschutzmittel als Bestandteil Polytetrafluorethylen (PTFE) enthält. Vorzugsweise sollte der Anteil des PTFE zwischen 0,01 - 12,00 Gew.-% (berechnet als Trockensubstanz) betragen. Das erfindungsgemäße Hautschutzmittel ist geeignet, die empfindliche, ekzembereite Haut zu schützen und außerdem zugleich auch eine Austrocknung der Haut zu verhindern, was insbesondere bei ekzembereiter Haut von wesentlicher Bedeutung ist. Nach der Applikation des erfindungsgemäßen Hautschutzmittels sind die manuellen Tätigkeiten und das Tastgefühl der das Hautschutzmittel benutzenden Personen nicht nennenswert beeinträchtigt; nach einer kurzen Antrocknungsphase bleibt auf der Haut kein fettiges Gefühl zurück. Weder der Wärme- noch der Gasaustausch werden durch die Anwendung des erfindungsgemäßen Hautschutzmittels spürbar beeinträchtigt. Die Hautschutzwirkung besteht über mehrere Stunden und wird selbst durch mehrmaliges Händewaschen nicht beeinträchtigt. Dank der glatten und geschmeidigen Oberfläche, die die Haut nach Anwendung des erfindungsgemäßen Hautschutzmittels erhält, wird die Rückbesiedelung der entsprechenden Hautpartien mit Bakterien deutlich verringert.

Die vorteilhafte Wirkung des erfindungsgemäßen Hautschutzmittels geht auf die im Hautschutzmittel enthaltene Komponente Polytetrafluorethylen (PTFE) zurück; diese Substanz ist bisher in Hautschutzmitteln nicht eingesetzt worden. Mittels des erfindungsgemäßen Hautschutzmittels wird auf der Haut ein sehr dünner Film geschaffen, der eine Semipermeabilität insofern gewährleistet, als von außen die Feuchtigkeit nicht durchdringt, während Schweiß von innen nach außen abdunsten kann.

Als besonders vorteilhaft hat sich ein Hautschutzmittel erwiesen, das 0,10 - 0,30 Gew.-% Glycerin, 2,00 - 4,00 Gew.-% Propylenglycol, 1,40 - 2,80 Gew.-% Cetearath-20, 0,50 - 1,50 Gew.-% Paraffin Oil, 0,50 - 1,00 Gew.-% Decyl Oleate, 2,70 - 5,50 Gew.-% Potassium Stearate, 1,90 - 3,90 Gew.-% TEA Stearate, 0,50 - 1,30 Gew.-% Glyceryl Stearate, 66,75 - 89,95 Gew.-% entmineralisiertes Wasser, 0,25 - 0,75 Gew.-% Cetearyl Alcohol, 0,10 - 12,00 Gew.-% PTFE und 0,10 - 0,20 Gew.-% Preservative enthält. Dieses Hautschutzmittel kann in Form einer semiliquiden Zubereitung aus einer Emulsion vorliegen.

Eine besonders wirksame Ausführungsform der Erfindung ergibt sich, wenn das Hautschutzmittel semiliquid zubereitet ist und 0,20 Gew.-% Glycerin, 3,00 Gew.-% Propylenglycol, 2,10 Gew.-% Cetearath-20, 1,00 Gew.-% Paraffin Oil, 0,75 Gew.-% Decyl Oleate, 4,10 Gew.-% Potassium Stearate, 2,90 Gew.-% TEA Stearate, 0,90 Gew.-% Glyceryl Stearate, 83,80 Gew.-% entmineralisiertes Wasser, 0,50 Gew.-% Cetearyl Alcohol, 0,60 Gew.-% PTFE und 0,15 Gew.-% Preservative enthält.

Ein zur Anwendung unmittelbar auf der Haut geeignetes salbenförmiges Hautschutzmittel mit besonders günstigen Eigenschaften hinsichtlich Verträglichkeit und Schutzwirkung gegen Feuchtigkeitsverlust und gegen die vorstehend genannten hautschädigenden Substanzen ergibt sich, wenn die im vorstehenden genannten Bereiche eingehalten werden, wobei statt 66,75 - 89,95 Gew.-% 62,00 - 87,45 Gew.-% entmineralisiertes Wasser, statt 0,25 - 0,75 Gew.-% 2,50 - 4,50 Gew.-% Cetearyl Alcohol und 0,25 - 1,00 Gew.-% Hydroxypropyl Guar und Hydroxypropyltrimonium Chloride vorgesehen sind.

Das salbenförmige Hautschutzmittel besitzt optimale Schutzeigenschaften, wenn es weitgehend die im vorstehenden genannten bevorzugtesten Einzelanteilswerte enthält, wobei statt 83,80 Gew.-% 80,30 Gew.-% entmineralisiertes Wasser, statt 0,50 Gew.-% 3,50 Gew.-% Cetearyl Alcohol und 0,50 Gew.-% Hydroxypropyl Guar und Hydroxypropyltrimonium Chloride enthält.

Bei weitgehend normaler, gesunder Haut können die vorstehend beschriebenen Hautschutzmittel ohne Einschränkung angewendet werden, wobei sie auch bei mehrmonatiger Anwendung zu keinen erkennbaren Unverträglichkeiten führen. Um die erfindungsgemäßen Hautschutzmittel auch bei Menschen mit empfindlicher, zur Austrocknung neigender Haut anwenden zu können und um Einsatzmöglichkeiten der erfindungsgemäßen Hautschutzmittel bei verschiedenen akuten und chronischen Dermatosen zu gewährleisten, ist es vorteilhaft, wenn das Hautschutzmittel statt 0,50 - 1,00 Gew.-% 1,00 - 2,00 Gew.-% Decyl Oleate, vorzugsweise 1,50 Gew.-% Decyl Oleate, und außerdem 1,00 - 2,00 Gew.-% Octyl Dodecanol, vorzugsweise 1,50 Gew.-% Octyl Dodecanol enthält. Der Anteil des entmineralisierten Wassers am Hautschutzmittel wird entsprechend reduziert.

Eine vorteilhafte Reduzierung des Reibwiderstands der Haut ergibt sich, wenn das Hautschutzmittel 0,01 - 2,00 Gew.-%, bevorzugt 0,05 Gew.-%, Isopropylmyristate enthält, wobei der Anteil des entmineralisierten Wassers entsprechend reduziert ist.

Bei bestimmten Anwendungsfällen ist es vorteilhaft, wenn das Hautschutzmittel besonders fetthaltig ist. Für derartige Anwendungsfälle empfiehlt es sich, den Anteil an Decyl Oleat um 5,00 Gew.-% zu erhöhen und einen zumindest 5,00 gew.-%igen Anteil bzw. einen um 5,00 Gew.-% erhöhten Anteil an Octyl Dodecanol vorzusehen und einen entsprechend reduzierten Anteil entmineralisierten Wassers vorzugeben.

Eine für die Anwendung des Hautschutzmittels besonders günstige Form ergibt sich, wenn die vorstehend genannten Bestandteile zu einem Anteil von 85 Gew.-% mit einem Anteil von 15 Gew.-% eines Gemisches aus Propan und Butan kombiniert werden, welches als Treibmittel dient. Das derartig zubereitete Hautschutzmittel kann in einfacher Weise in eine mit einem Ventil versehene Druckgaspackung eingebracht werden und als Schaum aus dieser Druckgaspackung austreten bzw. verwendet werden.

In einer weiteren, vorteilhaften Ausführungsform der Erfindung, die mit relativ geringem Aufwand herstellbar ist und besonders günstige Eigenschaften für den Benutzer aufweist, enthält das erfindungsgemäße Hautschutzmittel 0,50 - 2,00, vorzugsweise 1,00 Gew.-% (Gew.-% Wirkstofflösung) Decyl Oleate (Cetiol V), 0,50 - 2,00, vorzugsweise 1,00 Gew.-% Octyl Dodecanol (Eutanol G), 0,05 - 0,30, vorzugsweise 0,10 Gew.-% Polytetrafluorethylen (PTFE), 1,00 - 3,00, vorzugsweise 2,00 Gew.-% Glyceryl Stearate (Cutina MD), 3,00 - 5,00, vorzugsweise 4,00 Gew.-% Cetearyl Alcohol (Lanette O), 3,50 - 6,50, vorzugsweise 5,00 Gew.-% Stearic Acid (Stearin Siegert LUX), 0,50 - 1,50, vorzugsweise 1,00 Gew.-% Mineral Oil (Paraffin perliquid.), 0,50 - 1,50, vorzugsweise 1,00 Gew.-% Trilaureth-4-Phosphate (Hostaphat KW 340 N), 86,35 - 68,60, vorzugsweise 77,70 Gew.-% Water (Wasser entmineralisiert), 1,50 - 3,50, vorzugsweise 2,50 Gew.-% Propylenglycol (Propylenglykol), 1,50 - 3,50, vorzugsweise 2,50 Gew.-% Glycerine (Glycerin Ph.Eur.III 86%ig), 1,00 - 3,00, vorzugsweise 2,00 Gew.-% Sodium Cetylsarconisate (Medialan KF), 0,05 - 0,30, vorzugsweise 0,10 Gew.-% Allantoin (Allantoin), 0,05 - 0,30, vorzugsweise 0,10 Gew.-% Methyldibromo Glutaronitrile (und) Phenoxylethanol (Euxyl K 400). Die Einstellung des pH-Wertes von 7,8 - 8,0 erfolgt mit 2-Amino-2-Methyl-1-Propanol (AMP 95).

Das vorstehend beschriebene Hautschutzmittel wird mit einem Gewichtsprozentanteil von 91,00 Gew.-% Wirkstoff und einem Gewichtsprozentanteil von 9,00 Gew.-% Propan/Butan in Dosen abgefüllt, z.B. enthält eine 100 ml-Dose 82,80 g Wirkstoff und 8,20 g Propan/Butan.

## Patentansprüche

1. Hautschutzmittel zum Schutz der menschlichen Haut gegen Austrocknung und aggressive, hautirritierende und allergisierende Substanzen, welches in verschiedenen Darreichungsformen, z.B. als Gel, Lotion, Salbe, Creme, Schaum etc. ausbildbar ist, dadurch gekennzeichnet, daß es Polytetrafluorethylen (PTFE) enthält.

2. Hautschutzmittel nach Anspruch 1, dessen Anteil an PTFE 0,01 - 12,00 Gew.-% beträgt.

3. Hautschutzmittel nach Anspruch 2, das
0,10 - 0,30 Gew.-% Glycerin,
2,00 - 4,00 Gew.-% Propylenglycol,
1,40 - 2,80 Gew.-% Cetearath-20,
0,50 - 1,50 Gew.-% Paraffin Oil,
0,50 - 1,00 Gew.-% Decyl Oleate,
2,70 - 5,50 Gew.-% Potassium Stearate,
1,90 - 3,90 Gew.-% TEA Stearate,
0,50 - 1,30 Gew.-% Glyceryl Stearate,
66,75 - 89,95 Gew.-% entmineralisiertes Wasser,
0,25 - 0,75 Gew.-% Cetearyl Alcohol,
0,10 - 12,00 Gew.-% PTFE und
0,10 - 0,20 Gew.-% Preservative
enthält.

4. Hautschutzmittel nach Anspruch 3, das
0,20 Gew.-% Glycerin,
3,00 Gew.-% Propylenglycol,
2,10 Gew.-% Cetearath-20,
1,00 Gew.-% Paraffin Oil,
0,75 Gew.-% Decyl Oleate,
4,10 Gew.-% Potassium Stearate,
2,90 Gew.-% TEA Stearate,
0,90 Gew.-% Glyceryl Stearate,
83,80 Gew.-% entmineralisiertes Wasser,
0,50 Gew.-% Cetearyl Alcohol,
0,60 Gew.-% PTFE (berechnet als Trockensubstanz) und
0,15 Gew.-% Preservative
enthält.

5. Hautschutzmittel nach Anspruch 3, das
statt 66,75 - 89,95 Gew.-% 62,00 - 87,45 Gew.-% entmineralisiertes Wasser,
statt 0,25 - 0,75 Gew.-% 2,50 - 4,50 Gew.-% Cetearyl Alcohol und 0,25 - 1,00 Gew.-% Hydroxypropyl Guar und Hydroxypropyltrimonium Chloride
enthält.

6. Hautschutzmittel nach Anspruch 4, das
statt 83,80 Gew.-% 80,30 Gew.-% entmineralisiertes Wasser,
statt 0,50 Gew.-% 3,50 Gew.-% Cetearyl Alcohol und
0,50 Gew.-% Hydroxypropyl Guar und Hydroxypropyltrimonium Chloride
enthält.

7. Hautschutzmittel nach Anspruch 3, das
statt 0,50 - 1,00 Gew.-% 1,00 - 2,00 Gew.-% Decyl Oleate,
statt 66,75 - 89,95 Gew.-% 63,75 - 88,45 Gew.-% entmineralisiertes Wasser und
1,00 - 2,00 Gew.-% Octyl Dodecanol enthält.

8. Hautschutzmittel nach Anspruch 4, das
statt 0,75 Gew.-% 1,50 Gew.-% Decyl Oleate,
statt 83,80 Gew.-% 81,55 Gew.-% entmineralisiertes Wasser und
1,50 Gew.-% Octyl Dodecanol enthält.

9. Hautschutzmittel nach Anspruch 5, das
statt 0,50 - 1,00 Gew.-% 1,00 - 2,00 Gew.-% Decyl Oleate,
statt 62,00 - 87,45 Gew.-% 59,00 - 85,95 Gew.% entmineralisiertes Wasser und
1,00 - 2,00 Gew.-% Octyl Dodecanol
enthält.

10. Hautschutzmittel nach Anspruch 6, das
statt 0,75 Gew.-% 1,50 Gew.-% Decyl Oleate,
statt 80,30 Gew.-% 78,05 Gew.-% entmineralisiertes Wasser und
1,50 Gew.-% Octyl Dodecanol
enthält.

11. Hautschutzmittel nach einem der Ansprüche 1 - 10, das 0,01 - 2,00 Gew.-%, bevorzugt 0,05 Gew.-%, Isopropylmyristate und einen entsprechend reduzierten Anteil entmineralisierten Wassers enthält.

12. Hautschutzmittel nach einem der Ansprüche 1 - 11, das einen um 5,00 Gew.-% erhöhten Anteil an Decyl Oleat und einen zumindest 5,00 gew.-%igen Anteil bzw. einen um 5,00 Gew.-% erhöhten Anteil an Octyl Dodecanol und einen entsprechend reduzierten Anteil entmineralisierten Wassers enthält.

13. Hautschutzmittel nach einem der Ansprüche 1 - 12, das mit einem Anteil von 85,00 Gew.-% mit 15,00 Gew.-% eines Propan/Butan-Gemisches kombiniert ist.

14. Hautschutzmittel nach Anspruch 1, das
0,50 - 1,50 Gew.-% Decyl Oleate (Cetiol V),
0,50 - 1,50 Gew.-% Octyl Dodecanol (Eutanol G),
0,05 - 0,30 Gew.-% Polytetrafluorethylen (PTFE),
1,00 - 3,00 Gew.-% Glyceryl Stearate (Cutina MD),
3,00 - 5,00 Gew.-% Cetearyl Alcohol (Lanette O),
3,50 - 6,50 Gew.-% Stearic Acid (Stearin Siegert LUX),
0,50 - 1,50 Mineral Oil (Paraffin perliquid.),
0,50 - 1,50 Gew.-% Trilaureth-4-Phosphate (Hostaphat KW 340 N)
86,35 - 68,60 Gew.-% Water (Wasser entmineralisiert),
1,50 - 3,50 Gew.-% Propylenglycol (Propylenglykol),
1,50 - 3,50 Gew.-% Glycerine (Glycerin Ph.Eur.III 86%ig),
1,00 - 3,00 Gew.-% Sodium Cetylsarconisate (Medialan KF),
0,05 - 0,30 Gew.-% Allantoin (Allantoin) und
0,05 - 0,30 Gew.-% Methyldibromo Glutaronitrile (und) Phenoxylethanol (Euxyl K 400)
enthält.

15. Hautschutzmittel nach Anspruch 14, das
1,00 Gew.-% Decyl Oleate (Cetiol V),
1,00 Gew.-% Octyl Dodecanol (Eutanol G),
0,10 Gew.-% Polytetrafluorethylen (PTFE),
2,00 Gew.-% Glyceryl Stearate (Cutina MD),
4,00 Gew.-% Cetearyl Alcohol (Lanette O),
5,00 Gew.-% Stearic Acid (Stearin Siegert LUX),
1,00 Gew.-% Mineral Oil (Paraffin perliquid.),
1,00 Gew.-% Trilaureth-4-Phosphate (Hostaphat KW 340 N),
77,70 Gew.-% Water (Wasser entmineralisiert),
2,50 Gew.-% Propylenglycol (Propylenglykol),
2,50 Gew.-% Glycerine (Glycerin Ph.Eur.III 86%ig),
2,00 Gew.-% Sodium Cetylsarconisate (Medialan KF),
0,10 Gew.-% Allantoin (Allantoin) und
0,10 Gew.-% Methyldibromo Glutaronitrile (und) Phenoxylethanol (Euxyl K 400)
enthält.

16. Hautschutzmittel nach Anspruch 14 oder 15, bei dem der pH-Wert mittels 2-Amino-2-Methyl-1-Propanol (AMP 95) von 7,8 - 8,0 eingestellt ist.

17. Hautschutzmittel nach einem der Ansprüche 14 - 16, das mit einem Anteil von 91 Gew.-% Wirkstoff mit 9 Gew.-% eines Propan/Butan-Gemisches kombiniert ist.
